# EUROPEAN PATENT APPLICATION

(11) **EP 4 670 676 A1**
(43) Date of publication of application: **31.12.2025**
(21) Application number: 25183192.1
(22) Date of filing: 17.06.2025
(51) Int. Cl.: A61C 19/10

(54) **SHADE MATCHING AID FOR DENTAL SHADE GUIDE**

(30) Priority: 20.06.2024 JP 2024100036
(71) Applicant: Shofu Inc., Kyoto 605-0983 (JP)
(72) Inventor: TANAKA, Yosuke, Kyoto, 605-0983 (JP); SAKAGUCHI, Atsushi, Kyoto, 605-0983 (JP)
(74) Representative: Eisenführ Speiser

(57) **Abstract**

To provide a shade matching aid for shade guide that exhibits excellent shape adaptability to artificial tooth shade guides having various shapes and functions as a shade guide holder having high general versatility and gingiva color.

To provide a shade matching aid for shade guide for holding an artificial tooth shade guide with stick, comprising a main body including a surface having gingiva color, a recess for cervical portion configured to contact a cervical portion of an artificial tooth of an artificial tooth shade guide with stick, a stick insertion opening configured to be inserted with a stick of the artificial tooth shade guide with stick, a falling-off prevention mechanism configured to prevent falling-off of the artificial tooth shade guide with stick.

## Description

### BACKGROUND OF THE INVENTION

### Field of the Invention

The present invention relates to a shade matching aid for shade guide that holds a shade guide used in determining a shade of an artificial tooth or dental prosthesis device.

### Description of the Related Art

In dental clinics and dental laboratories, artificial tooth shade guides have been used for determining shades of artificial teeth and dental prosthesis devices (Japanese Unexamined Patent Application Publication No. 2006-6591). Specifically, the color, brightness, chroma and the like of a patient's intact tooth have been compared with artificial tooth shade guides, and a shade of the material to be used as the patient's teeth has been determined based on the obtained information (hereinafter referred to as shade taking).

However, in the case of determining a shade of a material based solely on a shade of an artificial tooth shade guide, as in the case of the artificial tooth shade guide of Japanese Unexamined Patent Application Publication No. 2006-6591, the background color is different from that of the oral cavity, therefore there has been a case where shade design is incorrect. Specifically, since a natural tooth in the oral cavity is in close contact with gingiva having red color, the shade, particularly near the cervical portion that is in close contact with gingiva, may appear different from the actual shade.

As one example of a solution to the problem in shade judgment caused by background color, Japanese Utility Model Registration No. 3036751 discloses a shade guide holder having gingiva color.

The shade guide holder having gingiva color disclosed in Japanese Utility Model Registration No. 3036751 has a recess for cervical portion that matches a shape of an artificial tooth of a artificial tooth shade guide, and a stick insertion opening that matches a shape of a stick of the artificial tooth shade guide, and by holding the artificial tooth shade guide during shade taking, it improves the reproducibility of color tone within the oral cavity.

### SUMMARY OF THE INVENTION

### Technical Problem

However, artificial tooth shade guides are currently sold by multiple manufacturers, and the shape of the artificial tooth and the shape of the stick vary greatly between shade guides from different manufacturers. Therefore, conventional shade guide holder having gingiva color is manufactured to fit only specific artificial tooth shape or stick shape, which vary by manufacturer. Thus, if the manufacturer is different, there is a case where the shade guide holder having gingiva color cannot hold the artificial tooth shade guide. Even if it is possible to hold, the artificial tooth of the artificial tooth shade guide and the recess for cervical portion of the shade guide holder having gingiva color are not morphologically compatible. Therefore, the artificial tooth of the held artificial tooth shade guide is likely to become displaced with respect to the recess for cervical portion of the shade guide holder having gingiva color, causing a problem that the artificial tooth shade guide falls off from the recess for cervical portion. Therefore, for example, if a dental clinic owns multiple types of artificial tooth shade guides, it needs to have a shade guide holder having gingiva color for each type of artificial tooth shade guide.

In addition, because it is require to share shade information between a dental clinic and a dental laboratory, a dental clinic and a dental laboratory are required to use a common artificial tooth shade guide. Therefore, for example, a dental clinic is required to have multiple types of artificial tooth shade guides for each dental laboratory. Thus, as the number of artificial tooth shade guides owned increases, it has been required to have many shade guide holders.

An object of the present invention is to provide a shade matching aid for shade guide that exhibits excellent shape adaptability to artificial tooth shade guides having various shapes and functions as a shade guide holder having high general versatility and gingiva color.

### Solution to Problem

The present invention provides a shade matching aid for shade guide for holding an artificial tooth shade guide with stick, comprising a main body including a surface having gingiva color, a recess for cervical portion configured to contact a cervical portion of an artificial tooth of an artificial tooth shade guide with stick, a stick insertion opening configured to be inserted with a stick of the artificial tooth shade guide with stick, a falling-off prevention mechanism configured to prevent falling-off of the artificial tooth shade guide with stick.

In the present invention, the stick insertion opening may be provided to be inclined with respect to the main body, and the falling-off prevention mechanism may be configured by the stick insertion opening.

In the present invention, a window may be formed below the recess for cervical portion.

In the present invention, a plurality of the windows may be formed below the recess for cervical portion, and a rib may be formed on a rear surface of the main body between adjacent windows.

In the present invention, the number of windows may be 3 to 6.

In the present invention, the insertion opening may be provided to be inclined at an angle of 1 to 5 ° with respect to the main body.

In the present invention, a dimension of the stick insertion opening in the front-back direction may be 0.4 to 1.2 mm.

In the present invention, the stick insertion opening may have a wide width section in which that a dimension in the front-back direction is expanded.

In the present invention, a plurality of ribs may be formed on a rear surface of the main body, and the falling-off prevention mechanism may be configured with the plurality of the ribs.

In the present invention, the plurality of the ribs may be configured so that a narrow width section in which a distance between the ribs may be narrowed is formed.

The present invention also provides a method of shade taking using an artificial tooth shade guide with stick inserted into the shade matching aid for shade guide according to the present invention.

The present invention also provides a set comprising the shade matching aid for shade guide according to the present invention and an artificial tooth shade guide with stick

In the present specification, the stick insertion opening is inclined with respect to the main body portion may mean that, in the side surface view of the shade matching aid for shade guide, the extension direction of the inner wall surface of the stick insertion opening is inclined with respect to the extension direction of the back surface of the main body.
In the present specification, the front side of the shade matching aid for shade guide may mean the front surface side of the main body.
In the present specification, the back side of the shade matching aid for shade guide may mean the rear surface side of the main body.
In the present specification, the front-back direction may mean the direction connecting the front side and the back side of the shade matching aid for shade guide.
In the present specification, the upper side of the shade matching aid for shade guide may mean the side on which the recess for cervical portion is formed.
In the present specification, the lower side of the shade matching aid for shade guide may mean the side opposite to the side on which the recess for cervical portion is formed.
In the present specification, the up-down direction may mean the direction connecting the upper side and the lower side of the shade matching aid for shade guide.
In the present specification, the left side of the shade matching aid for shade guide may mean the left side in a front view of the front surface side of the main body of the shade guide color matching aid from the front.
In the present specification, the right side of the shade matching aid for shade guide may mean the right side in a front view of the front surface side of the main body of the shade guide color matching aid from the front.
In the present specification, the left-right direction may mean the direction connecting the left side and the right side of the shade matching aid for shade guide.

### Advantageous Effects of Invention

According to the present invention, it is possible to provide a shade matching aid for shade guide that exhibits excellent shape adaptability to artificial tooth shade guides having various shapes and functions as a shade guide holder having high general versatility and gingiva color.

### Brief description of drawings

[FIG. 1] Front view of shade matching aid for shade guide according to one embodiment.
[FIG. 2] Rear view of shade matching aid for shade guide according to one embodiment.
[FIG. 3] Plan view of shade matching aid for shade guide according to one embodiment.
[FIG. 4] Bottom view of shade matching aid for shade guide according to one embodiment.
[FIG. 5] Right side view of shade matching aid for shade guide according to one embodiment.
[FIG. 6] Left side view of shade matching aid for shade guide according to one embodiment.
[FIG. 7] Front perspective view of shade matching aid for shade guide according to one embodiment.
[FIG. 8] Rear perspective view of shade matching aid for shade guide according to one embodiment.
[FIG. 9] Cross-sectional view along A-A line in Figure 1.
[FIG. 10] Schematic view of the front side of shade matching aid for shade guide in a state of holding two artificial tooth shade guides with stick.
[FIG. 11] Schematic view of the back side of shade matching aid for shade guide in a state of holding three artificial tooth shade guides with stick.
[FIG. 12] Diagram showing an example of the configuration of a plurality of ribs seen from the back side of the shade matching aid for shade guide.
[FIG. 13] Diagram showing an example of the configuration of a plurality of ribs seen from the back side of the shade matching aid for shade guide.
[FIG. 14] Diagram showing an example of the configuration of a plurality of ribs seen from the top side of the shade matching aid for shade guide.
[FIG. 15] Diagram showing an example of the configuration of a plurality of ribs seen from the top side of the shade matching aid for shade guide.
[FIG. 16] Diagram showing an example of the configuration of a plurality of ribs seen from the back side of the shade matching aid for shade guide.
[FIG. 17] Cross-sectional view of portion where stick insertion opening is formed.
[FIG. 18] Enlarged view of a part of figure 17.
[FIG. 19] Schematic view of side surface of shade matching aid for shade guide in a state of holding artificial tooth shade guide with stick.
[FIG. 20] Diagram showing example of commercially available artificial tooth shade guide with stick.
[FIG. 21] Diagram showing plan view of base formed with stick insertion opening and cross sectional view of main body.
[FIG. 22] Diagram showing bottom view of base formed with stick insertion opening and main body.
[FIG. 23] Diagrams showing modified examples of wide width section.
[FIG. 24] Diagrams showing modified examples of display position of shade symbol.

### DETAILED DESCRIPTION OF THE PREFERRED EMBODIMENTS

Hereinafter, specific embodiments of the present invention will be described in detail. However, the present invention is not limited to the following embodiment.

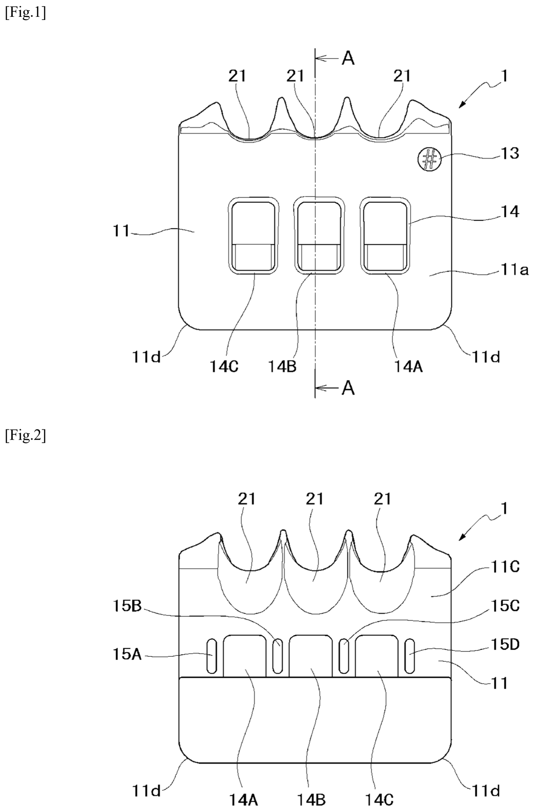

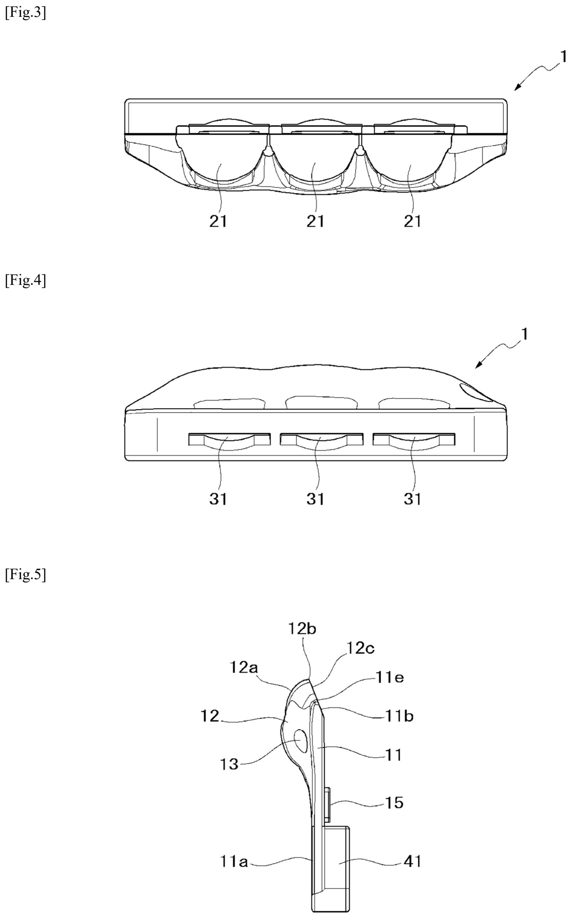

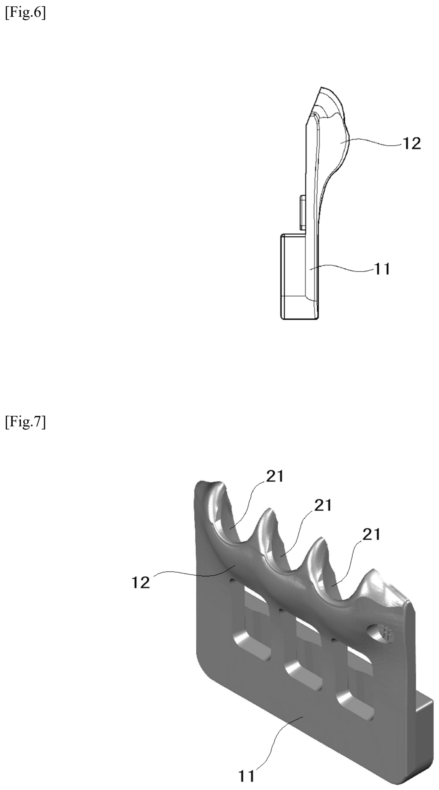

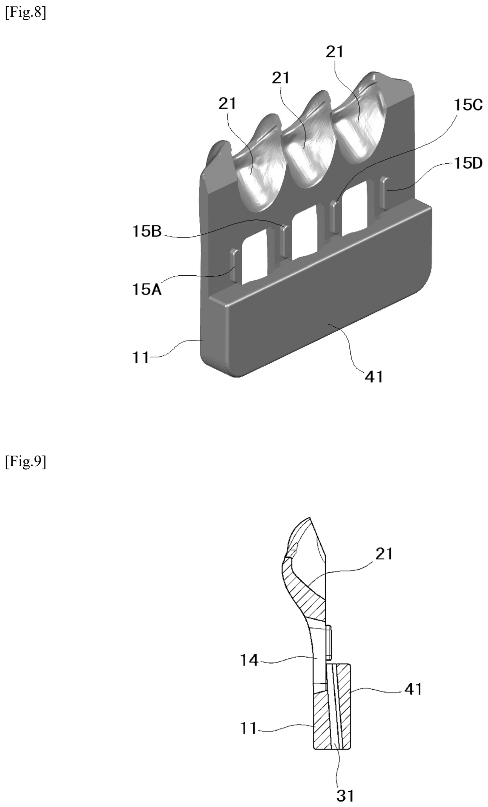

Figures 1 to 6 respectively show front view, rear view, plan view, bottom view, right side view, and left side view of a shade matching aid for shade guide 1 according to one embodiment of the present invention. Figures 7 and 8 respectively show a front perspective view and a rear perspective view of the shade matching aid for shade guide 1, and Figure 9 is a cross-sectional view taken along line A-A in Figure 1.

The shade matching aid for shade guide 1 of the present embodiment comprises a main body 11, a recess for cervical portion 21 that is configured to contact a cervical portion of an artificial tooth of an artificial tooth shade guide with stick 5 (described in detail below), and a stick insertion opening 31 that is configured to be inserted with a stick of an artificial tooth shade guide with stick 5. In the shade matching aid for shade guide 1 of the present embodiment, the recess for cervical portion 21 is formed at an upper end of the main body 11. The size of the shade matching aid for shade guide 1 may be, for example, 10 to 40 mm in height (the up-down direction in Figure 1), 20 to 120 mm in width (the left-right direction in Figure 1), and 2 to 10 mm in thickness (the front-back direction in Figure 1). The shade matching aid for shade guide 1 of the present embodiment has dimensions of 30 mm in height, 36 mm in width, and 5 mm in thickness. The number of the recess for cervical portion 21 and the stick insertion openings 31 may be set to the maximum number of the artificial tooth shade guide with stick to be held. The shade matching aid for shade guide 1 of the present embodiment has three shade matching aids for shade guide 21 and three stick insertion openings 31.

The main body 11 includes a front surface 11 a having gingiva color. The main body 11 of the present embodiment has a substantially rectangular shape in the front view shown in Figure 1 and is formed so as to have a constant thickness (the front-back direction in Figure 1). The main body 11 of the present embodiment further includes an upper surface 11b and a rear surface 11c. The main body 11 of the present embodiment has the upper surface 11b at a portion where the thickness (the front-back direction in Figure 1) gradually decreases. The rear surface 11c of the present embodiment is configured as a flat surface, and in the present embodiment, the extension direction of the rear surface 11c constitutes the extension direction of the rectangular shape of the main body 11. In the main body 11 of the present embodiment, both ends 11d in width direction of the lower portion in the front view shown in Figure 1 are round chamfered so as to be rounded. The value of the round chamfer is not particularly limited, but may be a radius of 0.2 to 5 mm. In particular, by setting the value of the round chamfer to 0.2 mm or more, it is possible to reduce pain even if the shade matching aid for shade guide accidentally comes into contact with a part of the patient's body in shade taking.

In the present embodiment, the main body 11 is provided with a raised portion 12 on the front surface 11a. The raised portion 12 is formed integrally with the main body 11 using the same material as the main body 11, and has gingiva color like the main body 11. The raised portion 12 in the present embodiment is provided from approximately the center of the height direction of the front surface 11a 11 to an upper end 11e of the top surface 11b, of the main body. The raised portion 12 in the present embodiment has a front surface 12a that constitutes the surface of the shade matching aid for shade guide 1 together with the front surface 11a of the main body 11, and a connecting surface 12c that connects an upper end 12b of the front surface 12a to the upper end 11e of the top surface 11b of the main body 11. In particular, in the present embodiment, the top surface 11b of the main body 11 and the connecting surface 12c are formed flush with each other so as to constitute the same plane. Therefore, in the present embodiment, the recess for cervical portion 21 is formed so as to extend across the upper end 11e of the main body 11 and the raised portion 12, and to open to the upper surface 11b of the main body 11 and the connecting surface 12c. The raised portion 12 in the present embodiment has a shape that resembles gingiva.

The material of the main body 11 is not particularly limited, but it is possible to manufacture from, for example, a resin material colored in gingiva color. Specifically, it can be manufactured from materials such as thermoplastic resins such as polyvinyl chloride, polystyrene, polyethylene terephthalate, polymethyl methacrylate, acrylonitrile-butadiene-styrene resin, and acrylonitrile-styrene resin; elastic materials such as polyethylene, polypropylene, and styrene-butadiene copolymer resin, and thermosetting resins such as polyurethane and silicone resin. In the present embodiment, the main body 11 is manufactured from acrylonitrile-styrene resin colored in gingiva color. Therefore, the main body 11 in the present embodiment has entirely gingiva color.

For the gingiva color of the main body 11, for example, the a* value, which is an index of redness, may be 5.0 or more. In the present invention, the "a* value" refers to one of the numerical values of color used in the L*a*b* color system, and the a* value indicates redness, that is, is the numerical value indicating the red direction of the L*a*b* color system chromaticity diagram. The a* value can be measured using a spectrophotometer, for example, by reflection colorimetry (measurement field of view: 2°, measurement diameter: 0.5 mm, light source: C) using a VSS7700 colorimeter (manufactured by NIPPON DENSHOKU INDUSTRIES Co.,Ltd). The main body 11 may be manufactured without limitation using a general manufacturing method of resin molding owned by the relevant manufacturer. As the general manufacturing method, injection molding, compression molding, extrusion molding, vacuum molding, build-up molding, and 3D printer modeling are used for manufacture. In the present embodiment, the main body 11 is manufactured by injection molding.

In the present embodiment, the recess for cervical portion 21 and the stick insertion opening 31 are formed integrally with the main body 11 using the same material as the main body 11. Therefore, in this embodiment, the recess for cervical portion 21 and the stick insertion opening 31 are also manufactured by injection molding from acrylonitrile-styrene resin colored in gingiva color, and have a gingiva color as a whole. However, the recess for cervical portion 21 and the stick insertion opening 31 may be manufactured using a material different from that of the main body 11 and may be manufactured as separate members.

In the main body 11 of the present embodiment, a shade symbol 13 indicating a shade of the gingiva color is displayed on the front surface 11a. Specifically, in the present embodiment, the shade symbol 13 is displayed near a window 14. By displaying such a shade symbol 13 on the front surface 11a, it becomes easier to manage the gingiva colored shade guide for each gingiva color. Further, it is possible to prevent incorrect selection of gingiva color. Such a shade symbol 13 may be displayed with a maximum dimension within a range of 2 mm to 5 mm, for example. Furthermore, the shade symbol 13 may be formed, for example, by imprinting a molding die with a maximum dimension of 3 mm in diameter near the raised portion 12 of the shade symbol.

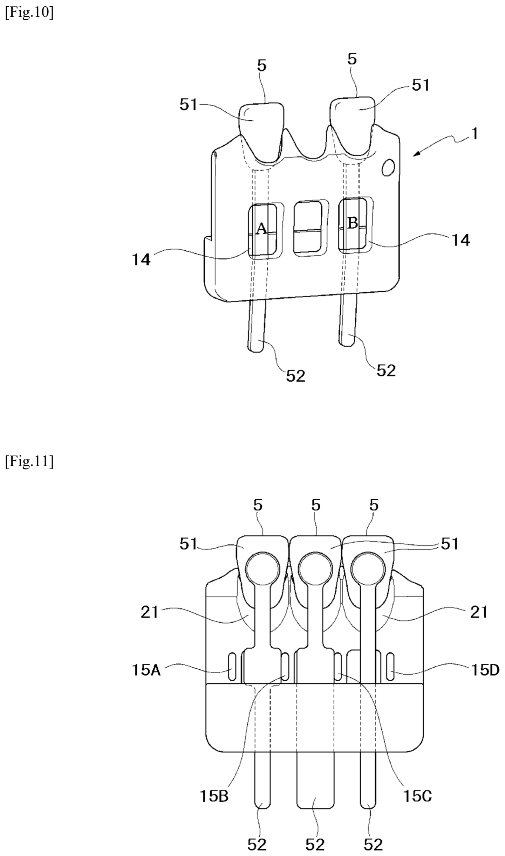

In the main body 11 of the present embodiment, the window 14 which penetrates the front surface 11a and the rear surface 11c is formed below the recess for cervical portion 21. In particular, the window 14 of the present embodiment is formed so that a portion thereof extends across the raised portion 12. For example, as shown in Figures 10 and 11, the window 14 may be formed in a position where a mark of the shade symbol (the "A" and "B" on the stick of the artificial tooth shade guide with stick 5 in Figure 10) displayed on the artificial tooth shade guide with stick 5 in a state that the artificial tooth shade guide with stick 5 is held can be seen from the front surface 11a side of the main body 11. By forming such a window, it is possible to check the mark of the shade symbol displayed on the artificial tooth shade guide with stick 5 without attaching and removing the artificial tooth shade guide with stick 5 from the shade matching aid for shade guide 1.

The window 14 may have a size that matches a size of the mark of the shade symbol on a commercially available artificial tooth shade guide with stick 5, and from the view point of general versatility, the height (the up-down direction in Figure1) may be 3 to 10 mm and the width (the left-right direction in Figure 1) may be 4 to 9 mm.

In particular, in the present embodiment, the shade symbol 13 is formed in the vicinity of the window 14. Therefore, it is possible to easily visually confirm the mark of the shade symbol of the artificial tooth shade guide with stick 5, which can be confirmed from the window 14, and the shade symbol 13 of the gingiva color at the same time. Thus, incorrect selection of gingiva color is effectively prevented. The shade symbol 13 may be formed in a location other than the vicinity of the window 14, or may be formed on a surface other than the front surface 11a of the main body 11.

In particular, in the present embodiment, the window 14 is formed so that it becomes larger from the rear surface 11c toward the front surface 11a. By forming the window in this manner, it is possible to easily visually confirm the mark of the shade symbol of the artificial tooth shade guide.

The number of the window 14, like the recess for cervical portion 21 and the stick insertion opening 31, may be set to the maximum number of the artificial tooth shade guide with stick to be held. In the present embodiment, three windows 14A to 14C are formed. The maximum number of the artificial tooth shade guide with stick to be held may be, for example, 1 to 10, or 3 to 6. By setting the maximum number of the artificial tooth shade guide with stick to be held to 3 or more, it is possible to easily consider the shade taking in a state of arranging a central incisor, a lateral incisor, and a canine. Furthermore, by setting the maximum number of the artificial tooth shade guide with stick to be held to 6 or less, it is possible to easily consider the shade taking for the six anterior teeth which are visible in the case of opening mouth. When a plurality of windows are formed, the distance between the windows can be determined by the dimension of the artificial tooth 51 of the artificial tooth shade guide with stick 5 to be held.

In the present embodiment, a plurality of ribs 15 are formed on the rear surface 11c of the main body 11. Specifically, the ribs 15A to 15D are formed on the rear surface 11c of the main body 11 so as to sandwich the plurality of the windows 14A to 14C. The ribs 15B and 15C are formed between adjacent windows 14. In the present embodiment, the plurality of the ribs 15 are formed integrally with the main body 11 using the same material as the main body 11.

The ribs formed between adjacent windows 14 function as a partition for adjacently held artificial tooth shade guides with stick 5 and also as a guide for the direction in which the artificial tooth shade guide with stick 5 is held. Therefore, in the present embodiment, by providing the ribs between adjacent windows 14, it is possible to hold the artificial tooth shade guide with stick 5 in the up-down direction regardless of the width of the stick of the held artificial tooth shade guide with stick 5. Thus, even if a large force in the left-right direction is applied to the shade matching aid for shade guide 1 holding the artificial tooth shade guide with stick 5, the movement of the stick of the artificial tooth shade guide with stick 5 in the left-right direction is restricted by the plurality of the ribs 15. Accordingly, the artificial tooth shade guide with stick 5 is prevented from falling off caused by large displacement of the stick of the artificial tooth shade guide with stick 5 in the left-right direction from the recess for cervical portion 21.

As in the present embodiment, the falling-off prevention mechanism that prevents the artificial tooth shade guide with stick 5 from falling off may be configured with the plurality of the ribs. However, if the falling-off prevention mechanism is configured by other means and the falling-off of the stick-attached artificial tooth shade guide 5 is sufficiently prevented, the plurality of the ribs 15 do not need to constitute the falling-off prevention mechanism, and even if the plurality of the ribs 15 are formed, there is no need to configure the falling-off prevention mechanism.

The shape and size of the plurality of the ribs 15 are not particularly limited, but for example, it is possible to be a vertically elongated approximately rectangular shape that is long in the up-down direction on the rear surface shown in Figure 2, having a horizontal width (dimension in the left-right direction) of 0.5 to 2 mm and a vertical width (dimension in the up-down direction) of 3 to 9 mm.

The material of the plurality of the ribs 15 is not particularly limited, but the same material as that used for the main body 11 may be used.

The plurality of the ribs 15 may be configured so that the distance between the plurality of the ribs 15 adjacent each other is constant or may be configured so that the distance between the plurality of the ribs 15 adjacent each other is variable. When the distance between the plurality of the ribs 15 adjacent each other is variable, for example, the plurality of the ribs 15 may be configured so that a narrow width section is formed where the distance between the ribs each other is narrowed.

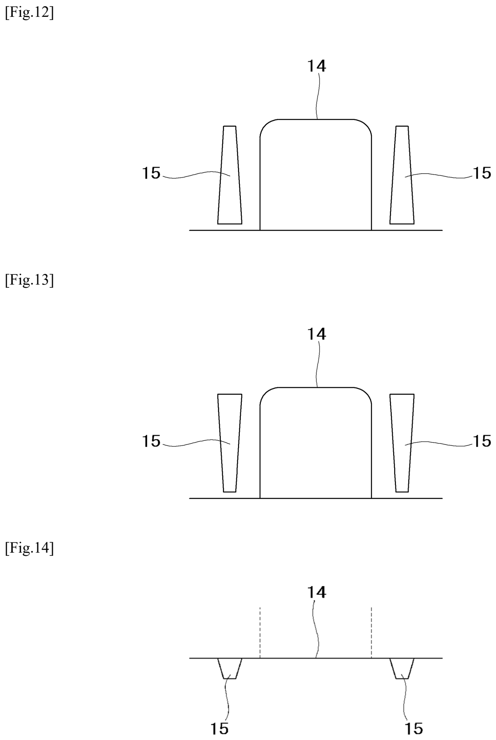

As shown in Figure 12, one example of the configuration of the plurality of the ribs 15 in the case of forming such narrow width section is a trapezoidal shape in which the lower side in the up-down direction of the plurality of the ribs 15 having the vertically elongated, approximately rectangular shape is increased to the left-right direction. In this case, it is possible to significantly prevent the displacement in the left-right direction around the artificial tooth of the artificial tooth shade guide with stick 5.

As shown in Figure 13, another example of the configuration of the plurality of the ribs 15 formed with the narrow width section is a trapezoidal shape in which the upper side n the up-down direction of the plurality of the ribs 15 having the vertically elongated, approximately rectangular shape is increased to the left-right direction. In this case, the plurality of the ribs 15 are formed along the stick that displaces in the left-right direction around the artificial tooth of the artificial tooth shade guide with stick 5, therefore it is possible to increase the amount of force absorbed by the plurality of the ribs 15.

As shown in Figure 14, the plurality of the ribs 15 may have a trapezoidal shape in which the front side dimension is larger than the back side dimension in the front-back direction of the plurality of the ribs 15. In this case, it is possible to easily replace the artificial tooth shade guide with stick 5.

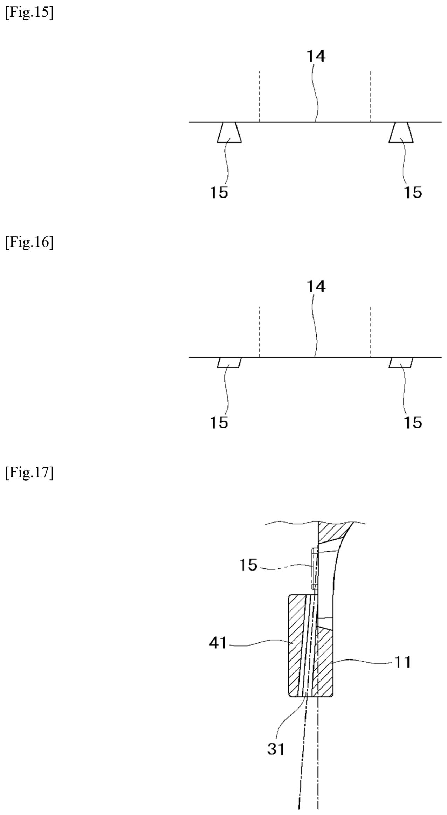

As shown in Figure 15, the plurality of the ribs 15 may have a trapezoidal shape in which the back side dimension is larger than the front side dimension in the front-back direction of the plurality of the ribs 15. In this case, it is possible to prevent the displacement of the artificial tooth shade guide with stick 5 in the front-back direction.

As shown in Figure 16, the plurality of the ribs 15 may have a parallelogram shape in which the back side in the front-back direction of the plurality of the ribs 15 is displaced in the left-right direction, in the plane view. In this case, it is possible to prevent the displacement of the stick around the artificial tooth of the artificial tooth shade guide with stick 5, while preventing the displacement of the artificial tooth shade guide with stick 5 in the front-back direction.

The plurality of the ribs 15 may not be in contact with the stick of the artificial tooth shade guide with stick 5 in a state of holding the artificial tooth shade guide with stick 5 in the up-down direction. In this case, it is possible to easily replace the artificial tooth shade guide with stick 5.

The plurality of the ribs 15 may be in contact with one of the left and right sides of the stick of the artificial tooth shade guide with stick 5 in a state of holding the artificial tooth shade guide with stick 5 in the up-down direction.

The plurality of the ribs 15 may be in contact with both the left and right sides of the stick of the artificial tooth shade guide with stick 5 in a state of holding the artificial tooth shade guide with stick 5 in the up-down direction.

In a state of holding the artificial tooth shade guide with stick 5 in the up-down direction, the recess for cervical portion 21 is in contact with at least a part of the cervical portion 51A of the artificial tooth 51 of the artificial tooth shade guide with stick 5. This recess for cervical portion 21 closely contacts the cervical portion of the artificial tooth to reproduce an appearance with no gaps, in order to mimic the close contact structure between a natural tooth and gingiva.

The recess for cervical portion 21 of the present embodiment is formed so as to extend across the upper end 11e of the main body 11 and the raised portion 12. Specifically, the recess for cervical portion 21 of the present embodiment is formed in a U-shape in the rear view as shown in Figure 2. In particular, the recess for cervical portion 21 of the present embodiment is formed so that the U-shape becomes smaller from the rear surface 11c side of the main body 11 toward the front surface 11a side.

The width of the recess for cervical portion 21 may be designed to fit a shape of the cervical portion 51A of the artificial tooth 51 of the commercially available artificial tooth shade guide with stick 5, but may be set to, for example, 7 to 10 mm, or 8 to 9 mm, from the viewpoint of general versatility. The inner surface of the recess for cervical portion 21 may be mirror-finished or textured.

The stick insertion opening 31 is configured to be inserted the stick of the stick-attached artificial tooth shade guide 5. In the present embodiment, the main body 11 has a base 41 below the rear surface side thereof, and the stick insertion opening 31 is formed on this base 41. Since the base 41 is formed integrally with the main body 11 using the same material as the main body 11, the stick insertion opening 31 is also formed integrally with the main body 11 using the same material as the main body 11.

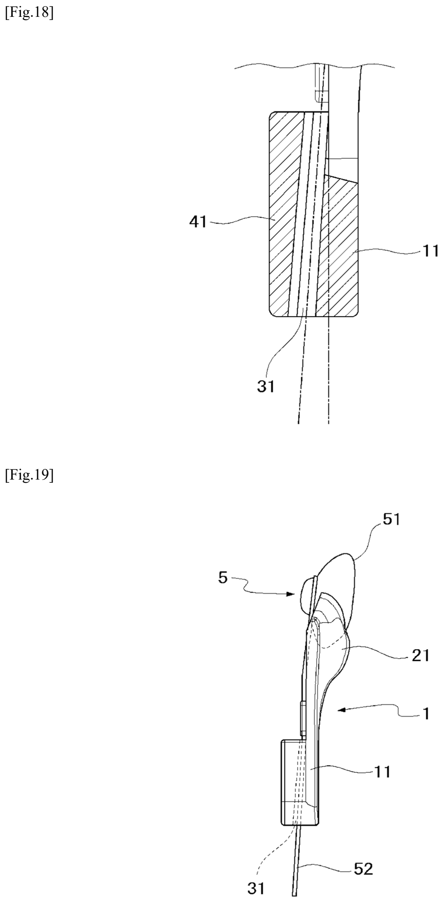

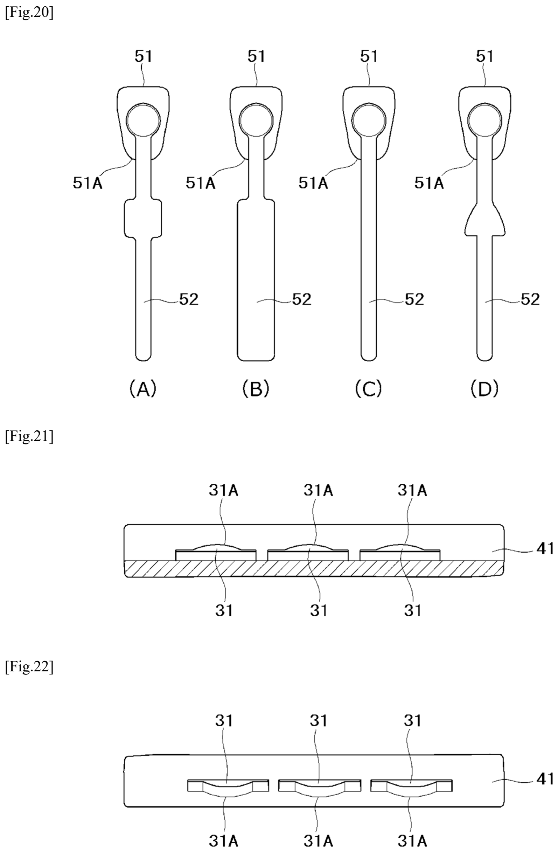

Figure 17 is a cross-sectional view of a portion where the stick insertion opening is formed. Figure 18 is an enlarged view of a part of figure 17. Figure 19 is a schematic view of the side surface of the shade matching aid for shade guide in a state of holding an artificial tooth shade guide with stick. Figures 20 (A) to (D) are diagrams showing an example of an artificial tooth shade guide with stick 5. As shown in detail in figures 17 to 19, the stick insertion opening 31 of the present embodiment is provided to be inclined with respect to the main body 11. Specifically, in the side surface view of the shade matching aid for shade guide 1, the extension direction of the stick insertion opening 31 is inclined with respect to the extension direction of the rear surface 11c of the main body 11, that is, with respect to the extension direction of the main body 11. In other words, in the side surface view of the shade matching aid for shade guide 1, the stick insertion opening 31 is not parallel to the main body 11. By such configuration of the stick insertion opening 31, in the stick 52 of the artificial tooth shade guide with stick 5 in a state of extending in the up-down direction due to its own weight, that is, extending parallel to the main body 11, a portion inserted in the stick insertion opening 31 is biased in a direction away from the main body 11 by contacting with a surface of the stick insertion opening 31 on the main body 11 side. Therefore, the stick 52 of the artificial tooth shade guide with stick 5 is curved as a whole so as to be convex toward the front surface side of the main body 11, and the artificial tooth 51 of the artificial tooth shade guide with stick 5 is biased so as to be pressed against the recess for cervical portion 21. Thus, the falling off the artificial tooth shade guide with stick 5 from the recess for cervical portion 21 is prevented, and therefore the tight fit between the recess for cervical portion 21 and the cervical portion 51A of the artificial tooth 51 is not impaired. Furthermore, since it is possible to generate the curvature of the stick 52 of the artificial tooth shade guide with stick 5 regardless of the shape of the artificial tooth shade guide with stick 5, it is possible to suppress the falling off of the artificial tooth shade guide with stick 5 from the recess for cervical portion 21 as long as the artificial tooth shade guide with stick 5 has a shape that allows it to be inserted into the stick insertion opening 31 regardless of the shape of the artificial tooth shade guide with stick.

As in the present embodiment, the falling-off prevention mechanism that prevents the falling off fo the stick-attached artificial tooth shade guide 5 may be configured by the stick insertion opening 31. However, if the falling-off prevention mechanism is configured by other means and the falling-off of the stick-attached artificial tooth shade guide 5 is sufficiently prevented, the stick insertion opening 31 does not need to constitute the falling-off prevention mechanism.

The stick insertion opening 31 may be provided to be inclined at an angle of 1 to 5 °, provided to be inclined at an angle of 3 to 5 °, with respect to the main body 11, for example. In the case of inclination angle of 1 ° or more, it is possible to further suppress the falling off the artificial tooth shade guide with stick 5 from the recess for cervical portion 21. In the case of inclination angle of 5 ° or less, it is possible to avoid that the stick of the artificial tooth shade guide with stick 5 cannot inserted into the stick insertion opening 31. When the surface facing the main body 11 and the surface opposite to the surface facing the main body 11 in the stick insertion opening 31 are not parallel in the side surface view of the shade matching aid for shade guide 1, it is possible to provide the surface facing the main body 11 of the stick insertion opening 31 to be inclined at an angle of 1 to 5 ° with respect to the main body 11, and 3 to 5 ° with respect to the main body 11.

Figure 21 is a diagram showing a plan view of the base 41 formed with the stick insertion opening 31 and a cross sectional view of the main body. Figure 22 is a diagram showing a bottom view of the base 41 formed with the stick insertion opening 31 and the main body. The stick insertion opening 31 may have a dimension of 0.4 to 1.2 mm in the front-back direction, for example. By having the dimension in the front-back direction of 0.4 mm or more, it is possible to avoid that the stick of the artificial tooth shade guide with stick 5 cannot inserted into the stick insertion opening 31. By having the dimension in the front-back direction of 1.2 mm or less, it is possible to easily hold a general artificial tooth shade guide with stick 5. The stick insertion opening 31 in the present embodiment has a dimension of 0.5 mm in the front-back direction.

The stick insertion opening 31 may have a dimension in the left-right direction of, for example, 4.5 to 10.0 mm. By having the dimension in the left-right direction of 4.5 mm or more, it is possible to avoid that the stick of the artificial tooth shade guide with stick 5 cannot inserted into the stick insertion opening 31. By having the dimension in the left-right direction of 10.0 mm or less, it is possible to easily hold a general artificial tooth shade guide with stick 5.

The stick insertion opening 31 may have a wide width section 31A in which the dimension in the front-back direction increases. By providing such a wide width section, it is possible to easily insert and remove a stick into the stick insertion opening 31.

The shape of the wide width section 31A in which the dimension of the stick insertion opening 31 in the front-back direction increases is not particularly limited, but the wide width section 31A may have a shape having an arc-shaped outline in the case of viewing from the bottom side of the base 41, as shown in Figure 21 and the like. The wide width section 31A may also have a shape having an elliptical arc-shaped outline in the case of viewing from the bottom side of the base 41, as shown in Figure 21 and the like.

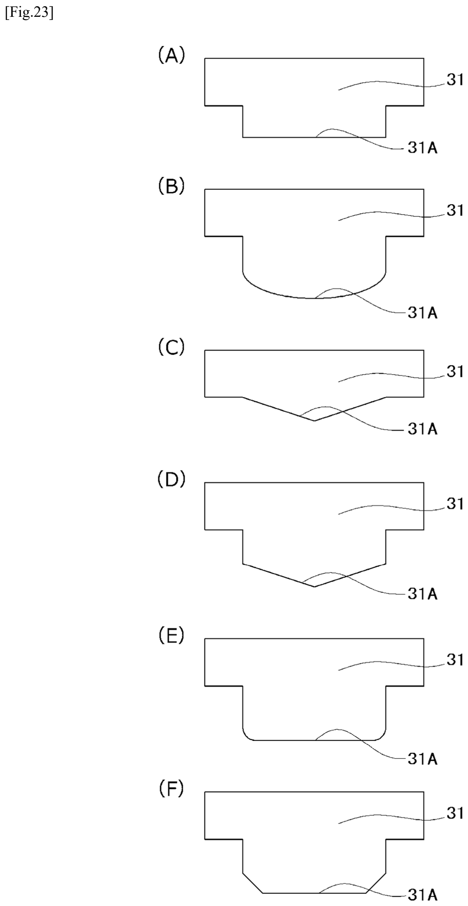

Figures 23 (A) to 23 (F) are diagrams showing modified examples of the wide width section 31A. As shown in Figure 23 (A), the wide width section 31 (A) may have a rectangular outline in the case of viewing from the bottom side of the base 41. The wide width section 31A may also have a trapezoidal outline in the case of viewing from the bottom side of the base 41.

As shown in Figure 23(B), in the case of viewing from the bottom side of the base 41, the wide width section 31A may have a shape that combines a portion having a shape of rectangular outline (or a portion having a shape of trapezoidal outline) that is continuous with the stick insertion opening 31 and a portion having a shape of arc-shaped outline (or a portion having an elliptical arc-shaped outline) that is continuous with the portion having a shape of rectangular outline.

As shown in Figure 23(C), in the case of viewing from the bottom side of the base 41, the wide width section 31A may have a shape having triangular outline. Further, as shown in Figure 23 (D), in the case of viewing from the bottom side of the base 41, the wide width section 31A may have a shape that combines a portion having a shape of rectangular outline (or a portion having a shape of trapezoidal outline) that is continuous with the stick insertion opening 31 and a portion having a shape of triangular outline that is continuous the portion having a shape of rectangular outline.

As shown in Figure 23 (E), in the case of viewing from the bottom side of the base 41, the wide width section 31A may have a shape of a rectangular outline in which both ends not connecting to the insertion opening 31 are round chamfered so as to be rounded. Further, as shown in Figure 23 (F), in the case of viewing from the bottom side of the base 41, the wide width section 31A have a shape of a rectangular outline in which both ends not connecting to the insertion opening 31 are tapered.

The dimension of the wide width section 31A in the front-back direction may be 0.5 to 2 times the dimension of the stick insertion opening in the front-back direction. In the present embodiment, the dimension in the front-back direction is 1 time (0.5 mm in the present embodiment).

For the stick insertion opening 31 in the base 41, for example, an upper end may contact the back surface of the main body 11 or may be positioned 0.5 to 3.0 mm from the back surface of the main body 11.

The base 41 may have any configuration as long as it can form the stick insertion opening 31. For example, the dimension of the base 41 in the front-back direction may be equal to or greater than, may be 1.5 times or more, or may be twice or more the dimension of the main body 11 in the front-back direction. For example, the dimension of the base 41 in the front-back direction may be 20 mm or less. By configuring the dimension in the front-back direction in this way, it is possible to place the shade matching aid for shade guide 1 in a state of freestanding (in a state of extending the main body 11 in the up-down direction).

In a state of holding the plurality of the artificial tooth shade guides with stick 5, the artificial teeth of the plurality of the artificial tooth shade guides with stick 5 may be in contact with each other or may be spaced apart by 0.1 to 0.5 mm.

The artificial tooth shade guide with stick 5 held by the shade matching aid for shade guide of the present embodiment may be a general artificial tooth shade guide with stick, and may be configured by fixing an artificial tooth 51 made of ceramic, glass ceramic, glass, or polymer material to a stick 52 made of metal or polymer material. Such general artificial tooth shade guide with stick 5 are sold in such a way that multiple are arranged horizontally, vertically, or in a fan shape and stored in a holder. When performing shade taking, the operator removes any artificial tooth shade guide with stick 5 from the holder and uses it.

In the case of the present embodiment, shade taking can be performed using the artificial tooth shade guide with stick inserted into the shade matching aid for shade guide of the present embodiment. Furthermore, the shade matching aid for shade guide of the present embodiment may constitute a set with an artificial tooth shade guide with stick that can be held by the shade matching aid for shade guide of the present embodiment.

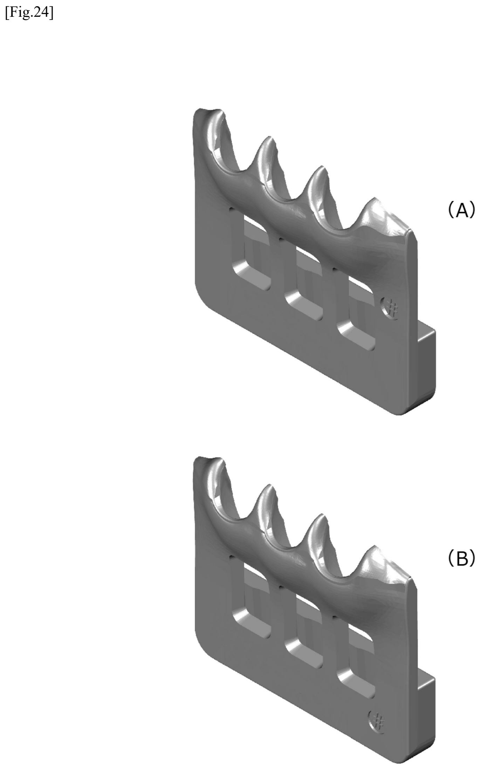

Figures 24 (A) to (B) are diagrams showing modified examples of display position of shade symbol 13. As shown in Figure 24 (A), the shade symbol 13 may be formed so that the top end of the shade symbol 13 and the top end of the window 14 are positioned at the same position in the up-down direction. Also, as shown in Figure 24 (B), the shade symbol 13 may be formed so that the top end of the shade symbol 13 is positioned lower than the bottom end of the window 14.

The shade matching aid for shade guide 1 for holding the artificial tooth shade guide with stick of the above embodiment comprises the main body 11 including the surface 11a having gingiva color, the recess for cervical portion 21 configured to contact the cervical portion 51A of the artificial tooth 51 of the artificial tooth shade guide with stick 5, the stick insertion opening 31 configured to be inserted with the stick 52 of the artificial tooth shade guide with stick 5, and the falling-off prevention mechanism configured to prevent falling-off of the artificial tooth shade guide with stick 5. Therefore, the shade matching aid for shade guide according to the above embodiment exhibits excellent shape adaptability to artificial tooth shade guides having various shapes and functions as a shade guide holder having high general versatility and gingiva color.

In the above embodiment, the stick insertion opening 31 is provided to be inclined with respect to the main body 11, and the falling-off prevention mechanism is constituted by the stick insertion opening 31.
By such configuration, in the stick 52 of the artificial tooth shade guide with stick 5 in a state of extending in the up-down direction due to its own weight, that is, extending parallel to the main body 11, a portion inserted in the stick insertion opening 31 is biased in a direction away from the main body 11 by contacting with a surface of the stick insertion opening 31 on the main body 11 side. Therefore, the stick 52 of the artificial tooth shade guide with stick 5 is curved as a whole so as to be convex toward the front surface side of the main body 11, and the artificial tooth 51 of the artificial tooth shade guide with stick 5 is biased so as to be pressed against the recess for cervical portion 21.
Thus, the falling off the artificial tooth shade guide with stick 5 from the recess for cervical portion 21 is prevented, and therefore the tight fit between the recess for cervical portion 21 and the cervical portion 51A of the artificial tooth 51 is not impaired. Furthermore, since it is possible to generate the curvature of the stick 52 of the artificial tooth shade guide with stick 5 regardless of the shape of the artificial tooth shade guide with stick 5, it is possible to suppress the falling off of the artificial tooth shade guide with stick 5 from the recess for cervical portion 21 as long as the artificial tooth shade guide with stick 5 has a shape that allows it to be inserted into the stick insertion opening 31 regardless of the shape of the artificial tooth shade guide with stick.

In the above embodiment, the window 14 is formed below the recess for cervical portion 21. Therefore, it is possible to check the mark of the shade symbol displayed on the artificial tooth shade guide with stick 5 without attaching and removing the artificial tooth shade guide with stick 5 from the shade matching aid for shade guide 1.

In the above embodiment, the plurality of the windows 14 are formed, and the plurality of the ribs 15 are formed on the rear surface 11c of the main body 11 between adjacent windows 14. Therefore, it is possible to hold the artificial tooth shade guide with stick 5 in the up-down direction regardless of the width of the stick of the held artificial tooth shade guide with stick 5. Thus, even if a large force in the left-right direction is applied to the shade matching aid for shade guide 1 holding the artificial tooth shade guide with stick 5, the movement of the stick of the artificial tooth shade guide with stick 5 in the left-right direction is restricted by the plurality of the ribs 15. Accordingly, the artificial tooth shade guide with stick 5 is prevented from falling off caused by large displacement of the stick of the artificial tooth shade guide with stick 5 in the left-right direction from the recess for cervical portion 21.

In the above embodiment, the number of the windows 14 is 3 to 6. Therefore, it is possible to easily consider the shade taking in a state of arranging a central incisor, a lateral incisor, and a canine and it is possible to easily consider the shade taking for the six anterior teeth which are visible in the case of opening mouth.

In the above embodiment, the stick insertion opening 31 is provided to be inclined at an angle of 1 to 5 ° with respect to the main body 11. Therefore, it is possible to suppress the falling off of the artificial tooth shade guide with stick 5 from the recess for cervical portion 21 and it is possible to avoid that the stick of the artificial tooth shade guide with stick 5 cannot inserted into the stick insertion opening 31.

In the above embodiment, the dimension of the stick insertion opening 31 in the front-back direction is 0.4 to 1.2 mm. Therefore, it is possible to avoid that the stick of the artificial tooth shade guide with stick 5 cannot inserted into the stick insertion opening 31 and to easily hold a general artificial tooth shade guide with stick 5.

In the above embodiment, the stick insertion opening 31 has the wide width section 31A in which the dimension in the front-back direction increases. Therefore, it is possible to easily insert and remove a stick into the stick insertion opening 31.

In the above embodiment, the plurality of the ribs 15 are configured to form the narrow width section where a distance between the ribs are narrowed.

As a method of shade taking using the shade matching aid for shade guide 1 of the above embodiment, for example, shade taking may be performed using an artificial tooth shade guide with stick inserted into a shade matching aid for shade guide.

The shade matching aid for shade guide 1 of the above embodiment may constitute a set with an artificial tooth shade guide with stick.

With respect to the use of substantially any plural and/or singular terms herein, those having skill in the art can translate from the plural to the singular and/or from the singular to the plural as is appropriate to the context.

Although the description herein has been given with reference to the drawings and embodiments, it should be noted that those skilled in the art may make various changes and modifications on the basis of this invention without difficulty. Accordingly, any such changes and modifications are intended to be included in the scope of the embodiments.

### Industrial applicability

According to the present invention, it is possible to provide a shade matching aid for shade guide that exhibits excellent shape adaptability to artificial tooth shade guides having various shapes and functions as a shade guide holder having high general versatility and gingiva color.

### Reference Signs List

1: shade matching aid for shade guide
11: main body
11a: front surface
11b: upper surface
11c: rear surface
11d: both ends
11e upper end
12: raised portion
12a: front surface
12b: upper end
12c: connecting surface
13: shade symbol
14: window
15: rib
21: recess for cervical portion
31: stick insertion opening
41: base
5: artificial tooth shade guide with stick
51: artificial tooth
51A: cervical portion
52: stick

## Claims

1. A shade matching aid for shade guide for holding an artificial tooth shade guide with stick, comprising
a main body including a surface having gingiva color,
a recess for cervical portion configured to contact a cervical portion of an artificial tooth of an artificial tooth shade guide with stick,
a stick insertion opening configured to be inserted with a stick of the artificial tooth shade guide with stick,
a falling-off prevention mechanism configured to prevent falling-off of the artificial tooth shade guide with stick.

2. The shade matching aid for shade guide according to claim 1, wherein
the stick insertion opening is provided to be inclined with respect to the main body, and
the falling-off prevention mechanism is configured by the stick insertion opening.

3. The shade matching aid for shade guide according to claim 2, wherein
a window is formed below the recess for cervical portion.

4. The shade matching aid for shade guide according to claim 3, wherein
a plurality of the windows are formed below the recess for cervical portion, and
a rib is formed on a rear surface of the main body between adjacent windows.

5. The shade matching aid for shade guide according to claim 4, wherein
the number of the windows is 3 to 6.

6. The shade matching aid for shade guide according to any one of claims 2 to 5, wherein the insertion opening is provided to be inclined at an angle of 1 to 5 ° with respect to the main body.

7. The shade matching aid for shade guide according to any one of claims 2 to 5, wherein a dimension of the stick insertion opening in the front-back direction is 0.4 to 1.2 mm.

8. The shade matching aid for shade guide according to any one of claims 2 to 5, wherein the stick insertion opening has a wide width section in which that a dimension in the front-back direction is expanded.

9. The shade matching aid for shade guide according to any one of claims 1 to 8, wherein
a plurality of ribs are formed on a rear surface of the main body, and
the falling-off prevention mechanism is configured with the plurality of the ribs.

10. The shade matching aid for shade guide according to claim 9, wherein
the plurality of the ribs are configured so that a narrow width section in which a distance between the ribs is narrowed is formed.

11. A method of shade taking using an artificial tooth shade guide with stick inserted into the shade matching aid for shade guide according to any one of claims 1 to 10.

12. A set comprising the shade matching aid for shade guide according to any one of claims 1 to10.
